# EUROPEAN PATENT APPLICATION

(11) **EP 4 667 009 A1**
(43) Date of publication of application: **24.12.2025**
(21) Application number: 23922837.2
(22) Date of filing: 29.09.2023
(51) Int. Cl.: A61K 33/40, A61K 9/06, A61K 9/127, A61K 31/7068, A61K 39/395, A61K 45/00, A61K 47/36, A61K 47/42, A61P 35/00, A61P 37/04, A61P 43/00

(54) **PHARMACEUTICAL COMPOSITION AND METHOD FOR ACTIVATING IMMUNITY AGAINST TUMOR**

(30) Priority: 17.02.2023 JP 2023023847
(71) Applicant: Kortuc Japan LLC, Tokyo 105-6004 (JP)
(72) Inventor: SUDA, Hiroyuki, Tokyo 105-6004 (JP); TOGASHI, Yosuke, Okayama-shi, Okayama 700-8530 (JP)
(74) Representative: Gulde & Partner
(86) International application number: PCT/JP2023/035757
(87) International publication number: WO 2024/171503

(57) **Abstract**

No method for treatment of distant tumor has been reported. A purpose of the present invention is to provide a pharmaceutical composition that is used in combination with radiotherapy to activate immunity against tumor, and that comprises a hydrogen peroxide solution. Another purpose of the present invention is to provide a method for activating immunity against tumor, the method comprising: a step for providing the pharmaceutical composition; a step for administering the pharmaceutical composition to a target site of a patient having the tumor; and a step for irradiating the target site with radiation. The tumor is distant from the target site of the patient having the tumor.

## Description

### [Technical Field]

The present invention relates to pharmaceutical compositions and methods for activating immunity against tumors when used in combination with radiation therapy. The compositions are characterized by containing hydrogen peroxide.

### [Background Art]

Radiation therapy is the second most common local treatment for malignant tumors after surgery, and the number of patients undergoing such therapy has been increasing dramatically because it is applicable to elderly patients and preserves normal organs and tissues. However, the high-energy X-rays and electron beams from linear accelerators currently used in radiation therapy are low linear energy transfer (LET) radiation, and their biological effects are relatively low. Therefore, linear accelerator radiation therapy is not effective enough for tumors such as malignant melanoma, various sarcomas and glioblastoma multiforme. In addition to malignant melanoma and various sarcomas, locally advanced cancers that have grown to several centimeters or more are radioresistant because of the large number of hypoxic tumor cells and the large amount of antioxidant enzymes they contain, making linear accelerator radiation therapy less effective. **In** addition, its effectiveness is limited to the localized tumor targeted by radiation therapy and cannot contribute to the therapeutic effect on patients with tumors in metastatic lesions.

In the report by Mole et al. in 1953, anti-tumor immunity induced by radiation therapy has also been discussed as an abscopal effect. Antitumor immunity has been reported as (1) activation of dendritic cells and T cells by Danger signals induced from dying cells, (2) induction of dendritic cells and activation of innate immunity associated with the activity of the type I interferon pathway, (3) induction of chemokines and adhesion factors involved in T cell migration and invasion, and (4) diversification of amino acid sequences of T cell receptors, suggesting that immunomodulatory mechanisms triggered by local irradiation are functioning. Therefore, control of distant metastasis may be possible if the abscopal effect can be induced at a high rate. However, it is extremely rare to encounter this abscopal effect with radiation therapy alone in actual clinical practice.

With the recent development of immune checkpoint inhibitors, the combined treatment of radiation therapy and immune checkpoint inhibitors has drawn increasing attention, by focusing on the abscopal effect of radiation therapy. Basic research is revealing that the combination therapy of radiation and immune checkpoint inhibitors such as anti-PD-L1 and anti-CTLA-4 antibodies induces a high rate of abscopal effect, suppresses distant metastasis, and prolongs survival. However, although several large-scale clinical trials have been conducted, their efficacy has been limited and unsatisfactory, and they have yet to be established as a new treatment modality. A more potent and safer method of activating tumor immunity is needed.

Various radiosensitizers have been developed to enhance the effects of radiation therapy (RT) (see, e.g., Patent Document 1). Hydrogen peroxide is one such radiosensitizer and has been reported to improve its anti-tumor effects by enhancing its radiosensitivity (Patent Document 1).

### [RELATED ART DOCUMENTS]

### [PATENT DOCUMENT]

[Patent Document 1] WO2008/041514

### [NON-PATENT DOCUMENT]

[Non-Patent Document 1] R.H. Mole, Br J Radiol. 1953 May;26(305):234-41

### [Disclosure of the Invention]

### [Problems to be Solved by the Invention]

The therapeutic effect of radiation is limited to the local area of treatment, and the use of radiosensitizers in general only enhances local effects. Satisfactory therapeutic effects on distant metastatic tumors have not been reported.

In addition, the combination therapy of radiation therapy and anti-tumor immunotherapeutic agents, such as immune checkpoint inhibitors, has not achieved a medically satisfactory therapeutic effect and has not yet become an established treatment method.

### [Means of Solving the Problems]

The inventors have found that a pharmaceutical composition containing a hydrogen peroxide solution, when used in combination with radiation, not only has a radiosensitizing effect but also activates immunity against tumors. Thereby, the inventors discovered that irradiation of a treatment site to which a pharmaceutical composition containing a hydrogen peroxide solution is administered not only improves the therapeutic effect locally where the radiation is administered, but also reduces the size of tumors located remotely from the treatment site. Based on these findings, the present invention was accomplished.

More surprisingly, the inventors found that the pharmaceutical composition containing hydrogen peroxide solution improves the effectiveness of combination therapy with radiation therapy and anti-tumor immunotherapeutic agents by activating tumor immunity.
[1] The purpose of the present invention is to provide a pharmaceutical composition including a hydrogen peroxide solution for activating immunity against one or more tumors when used in combination with radiation therapy.
   Radiotherapy using with the pharmaceutical composition of the present invention can reduce the size of one or more tumors located remotely from the treatment site by activating immunity against the one or more tumors.
[2] In the pharmaceutical composition according to [1], the one or more tumors may be located remotely from a target site of the patient having the one or more tumors.
[3] The pharmaceutical composition according to [1] or [2] may further include hyaluronic acid or salts thereof.
[4] The pharmaceutical composition of any one of [1] to [3] may further include liposomes, polymer gels, hydrogels or gelatin or salts thereof.
[5] The pharmaceutical composition of any one of [1] to [4] may be used in combination therapy with an anti-tumor immunotherapeutic agent.
[6] In the pharmaceutical compositions according to [5], the combination therapy may enhance one or more anti-tumor effects.
[7] In the pharmaceutical compositions according to [5] or [6], the anti-tumor immunotherapeutic agent may bind specifically to PD-1, PD-L1 or CTLA-4.
[8] In the pharmaceutical composition according to [7], the anti-tumor immunotherapeutic agent may be an immune checkpoint inhibitor.
[9] In the pharmaceutical composition according to [8], the immune checkpoint inhibitor may be a PD-1 inhibitor, PD-L1 inhibitor or CTLA-4 inhibitor.
[10] In the pharmaceutical composition according to [9], the immune checkpoint inhibitor may be atezolizumab, avelumab, durvalumab, ipilimumab, nivolumab or pembrolizumab or an antigen-binding fragment thereof.
[11] In the pharmaceutical composition according to [5] or [6], the anti-tumor immunotherapeutic agent may be an anti-tumor immunostimulatory chemotherapeutic agent.
[12] In the pharmaceutical composition according to [11], the anti-tumor immunostimulatory chemotherapeutic agent may be gemcitabine.
[13] In the pharmaceutical composition according to [5] or [6], the anti-tumor immunotherapeutic agent may be a tumor immune activating agent.
[14] The pharmaceutical composition according to [13], wherein the tumor immune activating agent may be a 41-BB agonist, an OX-40 agonist, a TIGIT inhibitor, a LAG-3 inhibitor or an IDO inhibitor.
[15] In the pharmaceutical composition according to [5] or [6], the above anti-tumor immunotherapeutic agent may be immune cells, nucleic acid molecules or a sensitizer.
[16] Another purpose of the present invention is to provide a method for activating immunity against one or more tumors, the method including:
   providing the pharmaceutical composition according to any one of [1] to [15];
   administering the pharmaceutical composition to a target site of a patient having the one or more tumors; and
   irradiating the target site with radiation,
   in which the one or more tumors are located remotely from the target site of the patient.
   The method according to the present invention can activate immunity against one or more tumors and reduce the size of the one or more tumors located remotely from the treatment site.
[17] The method according to [16] may further include administering an anti-tumor immunotherapeutic agent.

Since the method further inlclude the step of administering an anti-tumor immunotherapeutic agent, the method including the step, immunity against the one or more tumors can be activated and one or more anti-tumor effects can be enhanced in combination therapy with the anti-tumor immunotherapeutic agent.

### [Brief Description of the Drawings]

Fig. 1 shows a bar graph for the production of damage-associated molecular patterns (DAMPs), HMGB1, from cancer cells.
Fig. 2 shows a mouse illustrating the implantation site of MC38 cells and the site of the radiation therapy (RT) and an experimental schedule.
Fig. 3A shows a bar graph for the number of infiltrating cytotoxic T cells (CD8) of tumor-infiltrating lymphocytes (TILs) at the non-irradiated site, measured by flow cytometry.
Fig. 3B shows a bar graph for the number of infiltrating dendritic cells (DCs) in tumor-draining lymph nodes (TDLNs) near the irradiated site, measured using flow cytometry.
Fig. 4A shows a graph plotting tumor size at the irradiated site over time (n=6).
Fig. 4B shows a graph plotting tumor size at the non-irradiated site over time (n=6).
Fig. 5 shows a graph plotting tumor size at non-irradiated site over time (n 5).

### [Description of Embodiments]

### 1. Definition

For convenience, certain terms employed in the context of the present disclosure are collected here. Unless defined otherwise, all technical and scientific terms used herein have the same meaning as commonly understood by one of the ordinary skilled in the art to which this invention belongs. The singular forms "a", "an", and "the" are used herein to include plural referents unless the context clearly dictates otherwise.

Notwithstanding that the numerical ranges and parameters setting forth the broad scope of the invention are approximations, the numerical values set forth in the specific examples are described as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art.

The term "subject" or "patient" is used interchangeably herein and is intended to mean an animal including the human species that is treatable by the synthetic peptide and/or method of the present invention. The term "subject" or "patient" intended to refer to both the male and female gender unless one gender is specifically indicated. Accordingly, the term "subject" or "patient" comprises any mammal which may benefit from the treatment method of the present disclosure. Examples of a "subject" or "patient" include, but are not limited to, a human, rat, mouse, guinea pig, monkey, pig, goat, cow, horse, dog, cat, bird and fowl. In an exemplary embodiment, the patient is a human.

In this specification, unless otherwise specified, "% (w/v)" refers to a weight/volume percentage concentration.

Damage-associated molecular patterns (DAMPs) are biological substances released in response to cellular stress, such as cell death or cell damage, and function as alarm signals indicating cellular distress. In tumor immunity, DAMPs are released as an initial response involved in activating immune cells.

Hereinafter, embodiments of the present invention are illustrated in detail. The following embodiments are illustrative only and do not limit the scope of the present invention. In order to avoid redundancy, explanation for similar contents is not repeated.

### 2. Methods for activating immunity against tumors

The method for activating immunity against one or more tumors according to present embodiment includes the steps of providing a pharmaceutical composition containing a hydrogen peroxide solution, administering the pharmaceutical composition to a target site of a patient having the one or more tumors, and irradiating the target site with radiation, in which the tumor is located remotely from the target site of the patient having the one or more tumors.

### 3. Pharmaceutical compositions

The pharmaceutical composition for activating immunity against one or more tumors according to the present embodiment includes the hydrogen peroxide solution. The pharmaceutical composition according to the present embodiment can treat one or more tumors by activating immunity against the one or more tumors. The term "treat one or more tumors" or "one or more anti-tumor effects" means reducing the size of one or more tumors and/or inhibiting the growth of one or more tumors.

### 4. Hydrogen peroxide solution

The hydrogen peroxide solution according to the present embodiment is an aqueous solution containing hydrogen peroxide (H₂O₂; molecular weight: 34). The percentage of hydrogen peroxide in the pharmaceutical composition according to the present embodiment may be 0.01 to 3.5% (w/v), or may be within a range defined by any two values selected from the group consisting of 0.01%, 0.05%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, and 3.5% (w/v). Unless otherwise specified, the term "hydrogen peroxide solution" refers to a solution of hydrogen peroxide dissolved in distilled water, such as that specified in the Japanese Pharmacopoeia.

### 5. Additional Ingredients

The pharmaceutical composition may contain one or more additional ingredients. Examples of such additional ingredients include hyaluronic acid, liposomes, polymer gels, hydrogels, gelatin, or salts thereof. The additional ingredients may also include pharmaceutically acceptable physiological saline, buffers (e.g., phosphate buffer, Tris buffer, and acetate buffer), stabilizers, isotonic agents, and pH adjusters.

The ratio of hyaluronic acid or a salt thereof to hydrogen peroxide may be from 1 to 10,000 parts or within a range defined by any two values selected from the group consisting of 1, 5, 10, 50, 100, 150, 500, 1,000, 5,000, and 10,000 parts by mass of hyaluronic acid or a salt thereof (as total amount) per 100 parts by mass of hydrogen peroxide.

The hyaluronic acid according to the present embodiment may be extracted from animal tissues or produced by a fermentation method. The hyaluronic acid according to the present embodiment is preferably produced by the fermentation method. This is because hyaluronic acid produced by the fermentation method is safe and has high manufacturing stability. The strains used in the fermentation method include hyaluronic acid-producing microorganisms isolated from nature (e.g., Streptococcus sp.), Streptococcus equi FM-100 (FERM P 9027) described in Japanese Unexamined Patent Publication No. S63-123392, Streptococcus equi FM-300 (FERM P 2319) described in Japanese Unexamined Patent Publication No. H2-234689.

The hyaluronic acid according to the present embodiment has a mass average molecular weight of from 500,000 to 10,000,000, preferably from 500,000 to 8,000,000, and more preferably from 500,000 to 5,000,000.

The mass-average molecular weight of hyaluronic acid can be measured by the SEC-MALLS method, which uses size exclusion chromatograms (SEC) and a multi-angle light scattering (MALS) detector.

The hyaluronic acid according to the present embodiment may be used as an aqueous solution or a water-swelling gel.

The hyaluronic acids according to the present embodiment include non-cross-linked hyaluronic acid and cross-linked hyaluronic acid. The cross-linked hyaluronic acid is a polymer having a three-dimensional network structure. When the cross-linking points of cross-linked hyaluronic acid are cleaved, linear hyaluronic acid (non-cross-linked hyaluronic acid) is formed. The mass average molecular weight and degree of branching of the hyaluronic acid produced by cleaving the cross-linking points can be measured by GPC-MALS (multi-angle light scattering) using gel permeation chromatography (GPC), differential refractometry or a multi-angle laser light scattering (MALS) detector.

The hyaluronic acid according to the present embodiment may be non-cross-linked hyaluronic acid, cross-linked hyaluronic acid, or a combination thereof. The hyaluronic acid according to the present embodiment may be composed of different cross-linked hyaluronic acids and/or hyaluronic acids having different molecular weights. The hyaluronic acid according to the present embodiment may be a hyaluronate salt. The hyaluronate salt may be sodium hyaluronate, potassium hyaluronate or lithium hyaluronate.

The percentage of hyaluronic acid or salt thereof in the pharmaceutical composition may be from 0.1 to 10% (w/v) or may be within a range between any two values selected from the group consisting of 0.1, 0.5, 1.0, 2.0, 3.0, 4.0, 5.0, 6.0, 7.0, 8.0, 9.0, and 10% (w/v).

The isotonic agents include sodium chloride, glycerin, glucose, polyethylene glycol, propylene glycol, D-mannitol, fructose, xylitol, sodium dihydrogen phosphate, and sodium phosphate, preferably sodium chloride. The pH adjusters include hydrochloric acid and sodium hydroxide. The pH of the pharmaceutical composition according to the present embodiment is adjusted to a range of from 6 to 8.5, preferably from 6.8 to 7.8. The pharmaceutical composition according to the present embodiment may also contain a buffer for maintaining the pH.

### 6. Step of providing a pharmaceutical composition

The "the step of providing a pharmaceutical composition" according to the present embodiment may involve preparing or formulating a pharmaceutical composition. In one embodiment, the step of formulating a pharmaceutical composition includes mixing a hydrogen peroxide solution and hyaluronic acid or a salt thereof.

### 7. Step of administering the pharmaceutical composition to the target site of a patient having one or more tumors

The pharmaceutical composition according to the present embodiment is administered to a target site of a patient having one or more tumors. The method of local administration is not limited. In one embodiment, the target site is a site to whcih the pharmaceutical composition is administered and to which radiation is applied. **In** another embodiment, the target site is a site to which the pharmaceutical composition is administered, to which radiation is applied, and which contains one or more tumors requiring treatment.

The one or more tumors according to the present embodiment may be located remotely from the target site of the patient having the one or more tumors. In one embodiment, the one or more tumors are located remotely from the target site of the patient having the one or more tumors, and no detectable tumor is present at the target site. In another embodiment, the one or more tumors are located remotely from the target site of the patient having the one or more tumors and are also present at the target site. In yet another embodiment, the one or more tumor includes a first tumor and a second tumor, the first tumor(s) is/are located remotely from the target site of the patient having the one or more tumors, and the second tumor(s) is/are located at the target site of the patient having the one or more tumors. The first tumor(s) may be derived from a metastatic cancer, and the second tumor(s) may be derived from a primary cancer.

The term "tumor" refers to cells characterized by uncontrolled growth, and includes preneoplastic overgrowth, primary cancer, metastatic cancer, neoplasms, and solid tumors. The "tumor" may be caused by cancer. The cancer include, but are not limited to: lymphoma, squamous cell carcinoma, small cell lung cancer, non-small cell lung cancer, pulmonary adenocarcinoma, pulmonary squamous cell carcinoma, peritoneal cancer, adrenal cancer, anal cancer, bile duct cancer, bladder cancer, brain cancer, breast cancer, triple-negative breast cancer, central or peripheral nervous system cancer, cervical cancer, colon cancer, colorectal cancer, endometrial cancer, esophageal cancer, gallbladder cancer, gastrointestinal cancer, glioblastoma, head and neck cancer, renal cancer, hepatic cancer, nasopharyngeal cancer, nasal cavity cancer, oropharyngeal cancer, oral cancer, osteosarcoma, ovarian cancer, pancreatic cancer, parathyroid cancer, pituitary cancer, prostate cancer, retinoblastoma, sarcoma, salivary gland cancer, skin cancer, small intestine cancer, gastric cancer, testicular cancer, thymic cancer, thyroid cancer, uterine cancer, vaginal cancer, and vulvar cancer.

In one embodiment, the target tumor is a tumor caused by metastatic cancer and the non-target tumor is a tumor caused by primary cancer. In another embodiment, the target tumor is a tumor caused by primary cancer and the non-target tumor is a tumor caused by metastatic cancer.

### 8. Step of irradiating the target site with radiation

The "step of irradiating the target site with radiation" according to the present embodiment is performed after the "step of administering the pharmaceutical composition to the target site of a patient having one or more tumors." The pharmaceutical composition according to the present embodiment may activate immunity against the one or more tumors by irradiating the target site, to which the pharmaceutical composition has been administered, with radiation.

The radiation according to the present embodiment may be X-rays, electron beams, proton beams, heavy particle beams, α (alpha) rays, β (beta) rays, γ (gamma) rays, or a combination thereof. The X-rays or electron beams may be irradiated using a linear accelerator. Dose of the radiation according to the present embodiment may be from 1.5 to 4 Gy, or may be within a range between any two values selected from the group consisting of 1.5, 1.75, 2, 2.25, 2.5, 2.75, 3, 3.25, 3.5, 3.75, and 4 Gy. The irradiation according to the present embodiment may be performed 2 to 5 times per week, preferably 4 or 5 times per week. The irradiation according to the present embodiment may be conducted over a period of 1 to 5 weeks. Total dose of the radiation according to the present embodiment may be from 20 to 70 Gy or may be within a range between any two values selected from the group consisting of 20, 25, 30, 35, 40, 45, 50, 55, 60, 65, and 70 Gy.

### 9. Step of administering an anti-tumor immunotherapeutic agent

The pharmaceutical composition according to the present embodiment may be used in combination therapy with an anti-tumor immunotherapeutic agent. The method according to the present embodiment may further include the "step of administering the anti-tumor immunotherapeutic agent". In one embodiment, the "step of administering the anti-tumor immunotherapeutic agent" may be "step of administering the anti-tumor immunotherapeutic agent to the target site" or "step of administering the anti-tumor immunotherapeutic agent to a sit of the patient other than the target site".

The "step of administering the anti-tumor immunotherapeutic agent" may be performed after the "step of irradiating the target site with radiation", between the "step of administering the pharmaceutical composition to the target site of the patient having the one or more tumors" and the "step of irradiating the target site with radiation", or before the "step of administering the pharmaceutical composition to the target site of the patient having one or more tumors."

### 10. Anti-tumor immunotherapeutic agents

The anti-tumor immunotherapeutic agent according to the present embodiment refers to a substance that reduces tumor size or inhibits tumor growth by enhancing or supporting immune function against tumor. Such substances include, for example, compounds, cells (e.g., immune cells), proteins (e.g., antibodies), and nucleic acid molecules (e.g., DNA and RNA). The anti-tumor immunotherapeutic agents include anti-tumor immunostimulatory chemotherapeutic agents, immune checkpoint inhibitors, tumor immune activating agents, and combinations thereof.

In one embodiment, the anti-tumor immunotherapeutic agent may be an anti-tumor immunostimulatory chemotherapeutic agent (e.g., gemcitabine).

In one embodiment, the anti-tumor immunotherapeutic agent may specifically bind to PD-1, PD-L1, or CTLA-4. In another embodiment, the anti-tumor immunotherapeutic agent may be an immune checkpoint inhibitor. In one embodiment, the immune checkpoint inhibitor may be a PD-1 inhibitor, a PD-L1 inhibitor, or a CTLA-4 inhibitor. In another embodiment, the immune checkpoint inhibitor may be selected from atezolizumab, avelumab, durvalumab, ipilimumab, nivolumab, pembrolizumab, and an antigen-binding fragment of any of the foregoing.

In some embodiments, the anti-tumor immunotherapeutic agent may be a 41-BB agonist, an OX-40 agonist, a TIGIT inhibitor, a LAG-3 inhibitor or an IDO inhibitor.

### 11. Other embodiments

Provided also is a pharmaceutical composition for activating immunity against one or more tumors when used in combination with radiation therapy, (i) containing a hydrogen peroxide solution, which is used in combination therapy with a hydrogen peroxide solution and an anti-tumor immunotherapeutic agent, or (ii) containing an anti-tumor immunotherapeutic agent, which is used in combination therapy with a hydrogen peroxide solution and an anti-tumor immunotherapeutic agent.

Provided also is a pharmaceutical composition containing an anti-tumor immunotherapeutic agent, which is used in combination with a hydrogen peroxide solution and an anti-tumor immunotherapeutic agent to activate immunity against one or more tumors when used in combination with radiation therapy.

The use of a hydrogen peroxide solution in the manufacture of pharmaceutical compositions for activating immunity against one or more tumors is also provided. Use of hydrogen peroxide solution and anti-tumor immunotherapeutic agents in the manufacture of pharmaceutical compositions for activating immunity against one or more tumors is also provided.

### [EXAMPLES]

Data in the examples are expressed as the mean ± standard error of the mean (SEM). Statistical comparisons of the means were performed using Student's t-test. *P < 0.05, **P < 0.01, ***P < 0.001, ****P < 0.00001. "ns" indicates not significant.

### Example 1

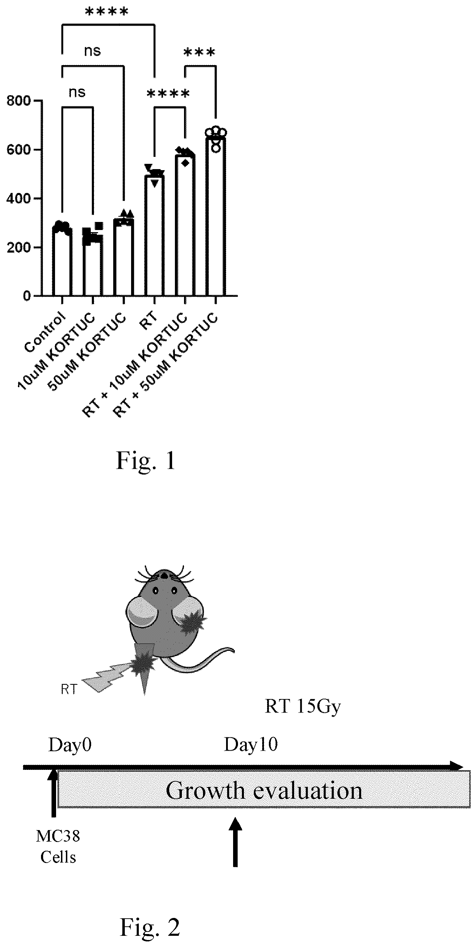

To determine whether the combination of hydrogen peroxide and radiation therapy (RT) induces the production of damage-associated molecular patterns (DAMPs), HMGB1, from cancer cells, KORTUC (a combination of hydrogen peroxide and hyaluronic acid) was added to MC38 cells (a mouse colon cancer cell line), followed by RT. The concentration of hydrogen peroxide in the culture medium was 0.0067% (w/v) or 0.0335% (w/v) (corresponding to 10 µM or 50 µM hydrogen peroxide), and the concentration of hyaluronic acid in the culture medium was 0.0111% (w/v) or 0.0556% (w/v). The RT dose was 5.0 Gy (1 Gy/min). A treatment in which neither KORTUC nor RT was administered was used as the control. Twenty-four hours after irradiation, the MC38 cells and culture supernatants were collected, and the HMGB1 levels were quantitatively measured by ELISA. The results are shown in Fig. 1.

Both KORTUC administration + RT (RT+KORTUC) and RT released HMGB1 into the culture supernatant. However, RT+KORTUC released a greater amount of HMGB1 into the culture supernatant than RT. These results suggest that the combination of KORTUC administration + RT may activate an immune response.

### Example 2

We investigated whether the combination of KORTUC (a mixture of hydrogen peroxide and hyaluronic acid; 0.5% hydrogen peroxide and 0.83% hyaluronic acid) administration and RT could induce an immune response in mice. As shown in Fig. 2, MC38 cells were implanted in the left leg (irradiated site) and the right abdomen (non-irradiated site) of mice on day 0. Ten days after cell implantation, the irradiated site was treated with 15 Gy of radiation (RT) or with KORTUC followed by 15 Gy of radiation (RT+KORTUC). The group receiving neither KORTUC nor RT was used as the control.

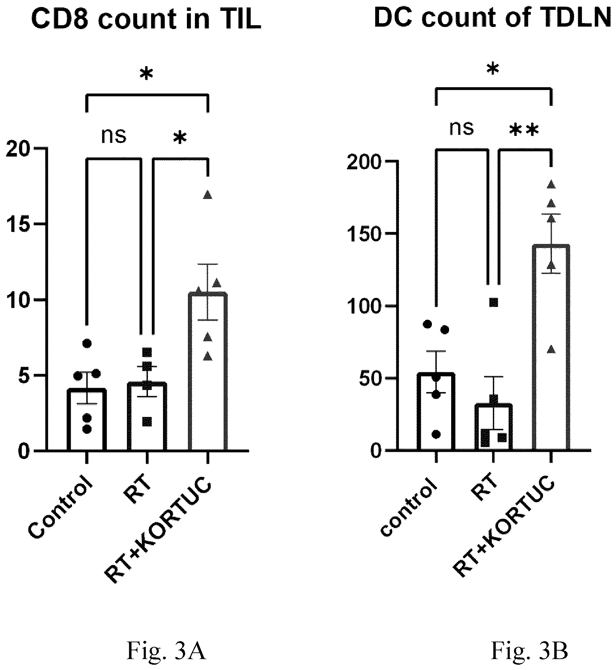

Fig. 3A shows a bar graph regarding the number of infiltrating cytotoxic T cells (CD8) of tumor-infiltrating lymphocytes (TILs) at the non-irradiated site, measured by flow cytometry. Fig. 3B shows a bar graph regarding the number of infiltrating dendritic cells (DCs) in tumor-draining lymph nodes (TDLNs) near the irradiated site, measured using flow cytometry.

RT+KORTUC significantly increased the number of infiltrating cytotoxic T cells (CD8) and dendritic cells (DCs) compared to the control and RT alone (Figs. 3A and 3B, respectively). These results demonstrate that RT+KORTUC activates an immune response in vivo. In particular, RT+KORTUC increased the number of cytotoxic T cells (CD8), which are immunocytes, at the non-irradiated site, indicating that RT+KORTUC is effective not only against local tumors but also against distant tumors.

### Example 3

The anti-tumor effects of the combination of KORTUC (a mixture of hydrogen peroxide and hyaluronic acid; 0.5% hydrogen peroxide and 0.83% hyaluronic acid) administration and RT was measured over time. MC38 cells were implanted on day 0 into the left leg (irradiated site) and right abdomen (non-irradiated site) of mice (see Fig. 2). Ten days after implantation, mice were treated with one of the following treatments at the irradiated site: administration of KORTUC (KORTUC alone), irradiation with 15 Gy of radiation alone (RT alone), or administration of KORTUC followed by irradiation with 15 Gy of radiation (KORTUC+RT). Mice receiving neither KORTUC nor radiation served as the control.

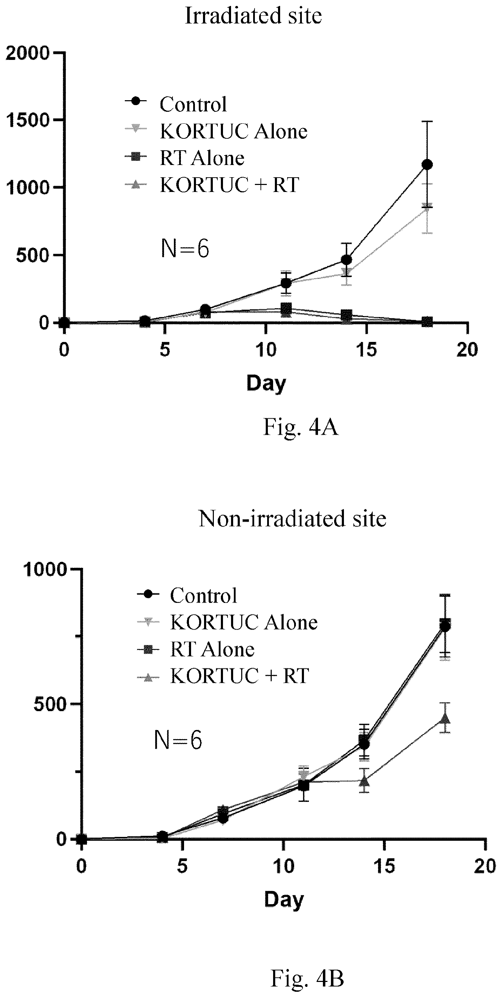

Fig. 4A shows a graph plotting tumor size at the irradiated site over time (n=6). Fig. 4B shows a graph plotting tumor size at the non-irradiated site over time (n=6). At the irradiated site, both RT alone and KORTUC+RT resulted in a time-dependent reduction in tumor size. At the non-irradiated site, KORTUC+RT showed an inhibitory effect on tumor growth. Although the one or more anti-tumor effects at the irradiated site were comparable between RT alone and KORTUC+RT, only KORTUC+RT significantly suppressed tumor growth at the non-irradiated site, while RT alone did not. This suggests that the observed effect is not due to a radiosensitizing effect of KORTUC at the irradiated site, but rather due to KORTUC-induced tumor immunity.

### Example 4

The effect of the combination of KORTUC (a mixture of hydrogen peroxide and hyaluronic acid; hydrogen peroxide concentration: 0.5%, hyaluronic acid concentration: 0.83%) administration, RT, and an anti-tumor immunotherapeutic agent was measured over time. MC38 cells were implanted in the left leg (irradiated site) and the right abdomen (non-irradiated site) of mice on day 0 (see Fig. 2). As the anti-tumor immunotherapeutic agent, an anti-PD-1 antibody (aPD1), which is an immune checkpoint inhibitor, was used. Ten days after implantation, mice were treated with one of the following treatments: intraperitoneal administration of aPD1 (aPD1 alone), intraperitoneal administration of aPD1 followed by irradiation with 15 Gy of radiation atthe irradiated site (RT + aPD1), or intraperitoneal administration of aPD1, administration of KORTUC followed by irradiation with 15 Gy of radiation at irradiated site (KORTUC + RT + aPD1). Mice receiving neither neither KORTUC, anti-PD-1 antibody, nor radiation served as the control.

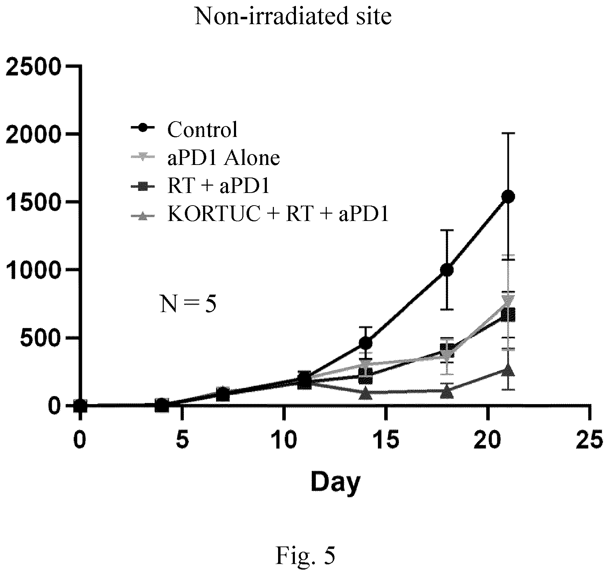

As shown in Fig. 5, tumor size reduced over time in the KORTUC + RT + aPD1 group. When comparing the results of KORTUC+RT in Fig. 4B with those of KORTUC+RT+aPD1 in Fig. 5, it is clear that KORTUC+RT+aPD1 exhibits a greater anti-tumor effect on distant tumors than KORTUC+RT.

It was demonstrated that the combination of KORTUC (hydrogen peroxide) administration and RT induced an immune response and showed one or more anti-tumor effects, particularly on distant tumors unrelated to the irradiated site or local injection. Furthermore, it was shown that the combination of KORTUC (hydrogen peroxide), RT, and administration of an anti-tumor immunotherapeutic agent induced a stronger immune response and showed a greater anti-tumor effect on distant tumors compared to the combination of KORTUC (hydrogen peroxide) and RT.

## Claims

1. A pharmaceutical composition for activating immunity against one or more tumors when used in combination with radiation therapy, comprising a hydrogen peroxide solution.

2. The pharmaceutical composition according to claim 1, wherein the one or more tumors are located remotely from a target site of the patient having the one or more tumors.

3. The pharmaceutical composition according to claim 1 or 2, further comprising hyaluronic acid or a salt thereof.

4. The pharmaceutical composition according to any one of claims 1 to 3, further comprising liposomes, polymer gels, hydrogels or gelatin or salts thereof.

5. The pharmaceutical composition according to any one of claims 1 to 4, used in combination therapy with an anti-tumor immunotherapeutic agent.

6. The pharmaceutical composition of claim 5, wherein the combination therapy enhances one or more anti-tumor effects.

7. The pharmaceutical composition according to claim 5 or 6, wherein the anti-tumor immunotherapeutic agent specifically binds to PD-1, PD-L1 or CTLA-4.

8. The pharmaceutical composition according to claim 7, wherein the anti-tumor immunotherapeutic agent is an immune checkpoint inhibitor.

9. The pharmaceutical composition according to claim 8, wherein the immune checkpoint inhibitor is a PD-1 inhibitor, PD-L1 inhibitor, or CTLA-4 inhibitor.

10. The pharmaceutical composition according to claim 9, wherein the immune checkpoint inhibitor is atezolizumab, avelumab, durvalumab, ipilimumab, nivolumab, or pembrolizumab or an antigen binding fragment thereof.

11. The pharmaceutical composition according to claim 5 or 6, wherein the anti-tumor immunotherapeutic agent is an anti-tumor immunostimulatory chemotherapeutic agent.

12. The pharmaceutical composition according to claim 11, wherein the anti-tumor immunostimulatory chemotherapeutic agent is gemcitabine.

13. The pharmaceutical composition according to claim 5 or 6, wherein the anti-tumor immunotherapeutic agent is a tumor immune activating agent.

14. The pharmaceutical composition according to claim 13, wherein the tumor immune activating agent is a 41-BB agonist, OX-40 agonist, TIGIT inhibitor, LAG-3 inhibitor or IDO inhibitor.

15. The pharmaceutical composition according to claim 5 or 6, wherein the anti-tumor immunotherapeutic agent is immune cells, nucleic acid molecules or a sensitizer.

16. A method for activating immunity against one or more tumors, the method comprising:
providing the pharmaceutical composition according to any one of claims 1 to 15;
administering the pharmaceutical composition to a target site of a patient having the one or more tumors; and
irradiating the target site with radiation,
wherein the one or more tumors are located remotely from the target site of the patient.

17. The method according to claim 16, further comprising administering an anti-tumor immunotherapeutic agent.
